(19) Europäisches Patentamt
European Patent Office
Office européen des brevets

(11) Veröffentlichungsnummer: **0 153 582**
**A2**

(12) # EUROPÄISCHE PATENTANMELDUNG

(21) Anmeldenummer: 85100619.7

(22) Anmeldetag: 23.01.85

(51) Int. Cl.⁴: C 07 F 7/22
A 01 N 55/04, A 01 N 55/08

(30) Priorität: 07.02.84 DE 3404253

(43) Veröffentlichungstag der Anmeldung:
04.09.85 Patentblatt 85/36

(84) Benannte Vertragsstaaten:
AT BE CH DE FR GB IT LI LU NL SE

(71) Anmelder: CHEMIE LINZ AKTIENGESELLSCHAFT
St. Peter-Strasse 25
A-4020 Linz(AT)

(84) Benannte Vertragsstaaten:
BE CH FR GB IT LI LU NL SE AT

(71) Anmelder: Lentia Gesellschaft mit beschränkter Haftung
Arabellastrasse 4 Postfach 81 05 08
D-8000 München 81(DE)

(84) Benannte Vertragsstaaten:
DE

(72) Erfinder: Saischek, Gerald, Dipl.-Ing.
Roseggerstrasse 4
A-4600 Wels(AT)

(72) Erfinder: Raml, Walter, Dr. Dipl.-Ing.
A-4202 Hellmonsödt 246(AT)

(72) Erfinder: Wittmann, Erwin, Dr.
Lustenauerstrasse 8
A-4020 Linz(AT)

(72) Erfinder: Bodingbauer, Hans, Dr.
Eben 72
A-4202 Hellmonsödt(AT)

(72) Erfinder: Graf, Josef
Kürnberg 116
A-4442 Kleinraming(AT)

(54) ## 1,3,2-Dioxaborinane.

(57) Neue 1,3,2-Dioxaborinane der allgemeinen Formel

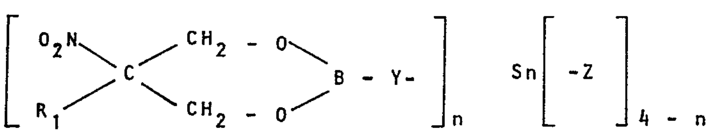

(I) ,

in welcher $R_1$ einen geradekttigen oder verzweigten Alkylrest mit 1 bis 20 Kohlenstoffatomen, einen Phenylrest oder Brom, Y einen 1-Oxy-2-thioäthylrest, einen 1-Oxy-4-thiophenylrest oder Sauerstoff, jeder Rest Z unabhängig von den anderen einen geradkettigen oder verzweigten Alkylrest mit 1 bis 20 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet, sowie Verfahren zu deren Herstellung. Die Verbindungen besitzen starke biozide Eigenschaften.

EP 0 153 582 A2

1,3,2-Dioxaborinane

Die Erfindung betrifft neue 1,3,2-Dioxaborinane, Verfahren zu deren Herstellung sowie diese enthaltende biozide Mittel, Verfahren zu deren Herstellung und deren Verwendung.

Es ist aus DE-PS 1 203 532 bekannt, daß Triphenylzinnverbindungen fungizide Eigenschaften aufweisen. Weiters ist aus DE-PS 21 15 666 bekannt, daß Trisneophylzinnverbindungen als akarizide Mittel verwendet werden können. Aus DE-PS 1 178 853 ist bekannt, daß borhaltige Organozinnmercaptoverbindungen als Stabilisatoren für Polyvinylchloridmassen, als Vulkanisations- oder Polymerisationsbeschleuniger geeignet sind.

Überraschenderweise konnte nun gefunden werden, daß bestimmte 1,3,2-Dioxaborinane, welche in Position 2 über den Rest Y mit einer Organozinnverbindung verbunden sind, hervorragende biozide Eigenschaften aufweisen.

Gegenstand der vorliegenden Erfindung sind demnach 1,3,2-Dioxaborinane der allgemeinen Formel

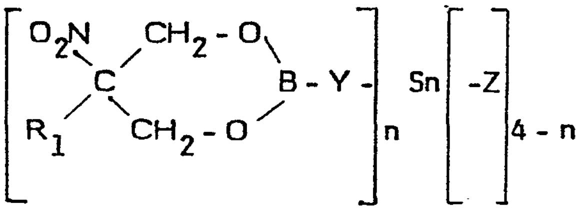

(I) ,

in welcher $R_1$ einen geradkettigen oder verzweigten Alkylrest mit 1 bis 20 Kohlenstoffatomen, einen Phenylrest oder Brom, Y einen 1-Oxy-2-thioäthylrest, einen 1-Oxy-4-thiophenylrest oder Sauerstoff, jeder Rest Z unabhängig von dem anderen einen geradkettigen oder verzweigten Alkylrest mit 1 bis 20 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet.

Weiterhin wurde gefunden, daß man 1,3,2-Dioxaborinane der allgemeinen Formel I erhält, wenn man ein Diol der allgemeinen Formel II

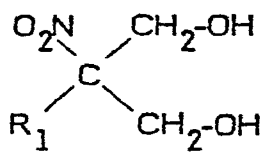

(II) ,

in welcher $R_1$ die oben angegebene Bedeutung hat, mit der äquivalenten Menge von Borsäure oder deren Anhydriden und gegebenenfalls mit der äquivalenten Menge von 2-Mercaptoäthanol oder von 4-Mercaptophenol bei einer Temperatur von 30 bis 180°C gegebenenfalls in einem unter Reaktionsbedingungen inerten Verdünnungsmittel unter Abspaltung von Wasser umsetzt und das Reaktionsprodukt, falls n = 1 ist, mit der äquivalenten Menge einer Verbindung der allgemeinen Formel

$$HO - Sn(-Z)_3 \qquad (III) ,$$

in welcher Z die oben angegebene Bedeutung hat oder deren Anhydrid und falls n = 2 ist, mit der äquivalenten Menge einer Verbindung der allgemeinen Formel

$$O = Sn(-Z)_2 \qquad (IV) ,$$

in der Z die oben angegebene Bedeutung hat, unter weiterer Wasserabspaltung bei einer Temperatur von 30 bis 180°C umsetzt und anschließend gegebenenfalls das inerte Verdünnungsmittel entfernt.

Die erfindungsgemäßen 1,3,2-Dioxaborinane zeigen starke biozide Eigenschaften gegen ein breites Spektrum von Schadorganismen und stellen somit eine Bereicherung der Technik dar.

In der Formel I steht der Rest $R_1$ zweckmäßig für einen Alkylrest mit 1 bis 15 Kohlenstoffatomen, insbesondere für einen Alkylrest von 1 bis 10 Kohlenstoffatomen. Besonders bevorzugt sind Alkylreste mit 1 bis 5 Kohlenstoffatomen sowie Brom. Der Rest Y steht insbesondere für Sauerstoff. Die Reste Z stehen zweckmäßigerweise für einen geradkettigen oder verzweigten Alkylrest von 1 bis 10 Kohlenstoffatomen, insbesondere für einen Alkylrest von 1 bis 5 Kohlenstoffatomen, wie etwa der Butyl- oder Neopentylrest. Für den Rest Z kommt weiters als Cycloalkyl-, Bicycloalkyl- oder Cycloalkylalkylrest etwa der Cyclohexyl-, der Norbornyl- oder der Cyclohexylmethylrest in Frage, als Phenylalkylrest etwa der Benzyl- oder der 2,2-Dimethyl-2-phenyläthylrest, der üblicherweise und auch im folgenden als "Neophyl"-rest bezeichnet wird. Neben dem Neophylrest sind der Cyclohexylrest und der Phenylrest besonders bevorzugt. Die Reste Z können gleich oder verschieden sein, bevorzugterweise sind sie gleich.

Die Herstellung der 1,3,2-Dioxaborinane der allgemeinen Formel I erfolgt zweckmäßigerweise, indem ein Mol eines Diols der allgemeinen Formel II mit einem Mol Borsäure, und falls Y nicht Sauerstoff bedeutet, mit einem Mol Borsäure und mit einem Mol 2-Mercaptoäthanol oder 4-Mercaptophenol zu den entsprechenden Dioxaborinanen umgesetzt wird, wobei die Mercaptoverbindung immer über die Hydroxylgruppe mit der Borsäure reagiert, und die Mercaptogruppe frei bleibt. Anschließend wird das Reaktionsprodukt, falls n = 1 ist, mit einem Mol einer Verbindung der allgemeinen Formel

$$HO - Sn(-Z)_3 \qquad (III) ,$$

in der Z die oben angegebene Bedeutung hat, oder mit 0,5 Mol einer Verbindung der allgemeinen Formel

$$O = Sn(-Z)_2 \qquad (IV) ,$$

in der Z die oben angegebene Bedeutung hat, umgesetzt.

Statt Borsäure ist es auch möglich, eines deren Anhydride, wie Metaborsäure oder Bortrioxyd, einzusetzen, ebenso können die organischen Hydroxyzinnverbindungen der allgemeinen Formel III auch als Anhydrid, als Bis-(Tri-organozinn)-oxid eingesetzt werden.

Die Reaktionsschritte können stufenweise durch Reaktion von Diol und Borsäure oder durch Reaktion von Diol, Borsäure und Hydroxy-mercaptoverbindung und anschließende Reaktion mit der Zinnverbindung durchgeführt werden. Wird keine Hydroxymercaptoverbindung in das Molekül eingebaut, dies ist der Fall, wenn Y Sauerstoff bedeutet, so ist es besonders vorteilhaft, sämtliche Reaktionspartner gleichzeitig miteinander reagieren zu lassen.

Vorteilhafterweise erfolgt die Reaktion in einem für die Reaktion inerten Lösungs- oder Verdünnungsmittel etwa bei Temperaturen zwischen 30 und 180$^0$C, vorzugsweise zwischen 60 und 120$^0$C bei Atmosphärendruck oder leicht reduziertem Druck. Als Verdünnungsmittel eignen sich beispielsweise halogenierte oder nicht halogenierte, aliphatische oder aromatische Kohlenwasserstoffe, wie Benzinfraktionen, Benzol, Chlorbenzole, Toluol, Xylole, Chloroform, Methylenchlorid.

Da die Gesamtreaktion unter Wasserabspaltung erfolgt, kann das Reaktionsende leicht an der Menge des entstandenen Reaktionswassers festgestellt werden, das aus der Reaktionsmischung etwa durch Azeotropdestillation entfernt wird. Nach beendeter Reaktion wird das Verdünnungsmittel durch Destillation bei Atmosphärendruck oder besonders vorteilhaft unter vermindertem Druck entfernt, worauf meist ein viskoser, öliger Rückstand entsteht, der ohne weitere Reinigungsschritte weiterverwendet werden kann.

Das Reaktionsende und der vollständige Umsatz zur gewünschten Endverbindung sind auch aus spektroskopischen Daten ersichtlich: Im Infrarotspektrum verschwinden die Adsorbtionen im Bereich von 650 bis 800 $cm^{-1}$ (Sn-O-Sn-Bindung) und im Bereich von 3300 bis 3700 $cm^{-1}$ (freie bzw.assoziierte Streckschwingungen). Die Bildung des Dioxaborinanringes wird durch das $^1$H-NMR-Spektrum (100 MHz) charakterisiert, wobei die Methylenprotonen des Ringes ein typisches AB-System mit einer Kopplungskonstante von J = 12 Hz zeigen.

Austauschbare Protonen sind nicht mehr vorhanden.

Zur Charakterisierung dienten weiters:

$z = \frac{f_A + f_B}{2}$ : Zentrum der Aufspaltung des AB-Systems der Methylenprotonen im 1,3,2-Dioxaborinanring (in ppm)

$f_A - f_B$: Differenz der Resonanzfrequenzen des AB-Systems (in Hz).

Die erfindungsgemäßen Verbindungen können zur Behandlung von technischen Materialien und Pflanzen eingesetzt werden, die durch die Einwirkung von Algen, Bakterien, Pilzen oder Insekten erkrankt sind oder vor diesen geschützt werden sollen. Die Mittel können aufgrund ihrer fungiziden und algiziden Wirksamkeit zur Bekämpfung von Schadorganismen aus den Klassen der Schizomyceten, Myxomyceten, Phycomyceten, Ascomyceten, Basidiomyceten und Deuteromyceten, und zur Bekämpfung von Algen eingesetzt werden. Zur Bekämpfung von Insekten und Milben, insbesondere der Ordnung Acarina, eignen sich vor allem jene erfindungsgemäßen Mittel, in denen die Reste $R_1$ und Y sowie die Zahl n die oben angegebene Bedeutung haben, und jeder Rest Z unabhängig von den anderen einen geradkettigen oder verzweigten Alkylrest mit 1 bis 20 Kohlenstoffatomen, einen Cycloalkyl-, Bicycloalkyl-, Cycloalkylalkyl- oder einen Phenylalkylrest bedeutet. Als Insekten gelten z.B. Insekten der Ordnung Lepidoptera, Coleoptera, Neuroptera, Rhynchota, Copeognatha, Diptera, Thysanoptera, Orthoptera, Anoplura und Hymenoptera. Es können sowohl pflanzenschädigende Milben, z.B. der Familien Tetranychidae, Tarsonemidae, Eryophyidae, Tyroglyphidae und Glycyphagidae, als auch ectoparasitäre Milben und Zecken, z.B. der Familien Ixodidae, Argasidae, Sarcoptidae und Dermanyssidae hervorragend bekämpft werden.

Die gute Pflanzenverträglichkeit und die zum Teil systemische Wirksamkeit in den zur Behandlung von Pflanzenkrankheiten notwendigen Konzentrationen erlaubt eine Behandlung von oberirdischen Pflanzenteilen, von Pflanz-und Saatgut und des Bodens. Als Pflanzen seien etwa Getreidearten, wie Weizen, Gerste, Roggen oder Hafer, Reis, weiters Mais, Baumwolle, Soja, Kaffee, Zuckerrohr, Zuckerrüben oder Kürbisgewächse und Kohlarten genannt.

Mit besonders gutem Erfolg können die erfindungsgemäßen Verbindungen etwa zur Bekämpfung folgender Pflanzenkrankheiten eingesetzt werden:

| | |
|---|---|
| Erysiphe graminis | an Getreide |
| Erysiphe betae | an Zuckerrüben |
| Podosphaera leucotricha | an Äpfeln |
| Uncinula necator | an Wein |
| Puccinia-Arten | an Getreide |
| Ustilago-Arten | an Getreide |
| Venturia inaequalis | an Äpfeln |
| Septoria nodorum | an Weizen |
| Septoria apii | an Sellerie |
| Cercospora beticola | an Zuckerrüben |
| Phytophthora infestans | an Kartoffel und Tomate |
| Peronospora viticola | an Wein |
| Alternaria solani | an Kartoffeln |
| Helminthosporium avenae | an Gerste |
| Tilletia caries | an Weizen |

In entsprechenden Aufwandmengen können die Stoffe auch als Holzschutzmittel gegen holzverfärbende Pilze und Moderfäulepilze eingesetzt werden, wie z.B. zur Bekämpfung von Merulius lacrimans, Polyporus vaporarius, Poria placenta, Polystictus cinnabarinus. Die erfindungsgemäßen Mittel können weiters zur Algen- und Schleimbekämpfung in landwirtschaftlichen Kulturen mit Überflutung, bei Industrieprozessen, in Kühlanlagen und Abwässern, in Gewächshäusern, Gewässern, Schwimmbecken und Aquarien oder als Zusätze zu Bau- und Kunststoffen, Leimen, Lacken oder anderen Anstrichmitteln verwendet werden.

Die Wirkstoffe können je nach ihrem Anwendungsgebiet in die üblichen Formulierungen übergeführt werden, wie Lösungen, Spritzpulver, Emulsionskonzentrate, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in

Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden, verflüssigten Gasen und/oder festen Trägerstoffen, gegebenenfalls unter Verwendung von Tensiden, also Emulgatoren und/oder Dispergier- und/oder Netzmitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxyd, sowie Wasser. Mit verflüssigten, gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe, sowie Butan, Propan, Stickstoff und Kohlendioxyd; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Produkte, wie hochdisperse Kieselsäure, Aluminiumoxyd und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen, sowie Granulate aus organischem Material, wie Sägemehl, Kokosnußschalen, Maiskolben und Tabakstengel; als Emulgiermittel und/oder schaumerzeugende Mittel kommen in Frage: z.B. nichtionogene und ionogene Tenside, wie Polyoxyäthylen-Fettsäure-Ester, Polyoxyäthylen-Fettalkoholäther, z.B. Alkylarlypolyglykoläther, Alkylsulfonate, Alkylsulfate, Arylsulfonate und Arylalkylsulfonate sowie Eiweißhydrolysate; als Dispergiermittel kommen in Frage z.B. Ligninsulfonate oder Kondensationsprodukte von Arylsulfonaten mit Formaldehyd.

Es können in den Formulierungen Haft- und Verdickungsmittel, wie Carboxymethylcellulose, Methylcellulose, natürliche und synthetische pulverige, körnige und latexförmige Polymere, wie Gummiarabicum, Polyvinylalkohol, Polyvinylacetat verwendet und anorganische oder organische Farbstoffe zugesetzt werden.

Die Formulierungen enthalten im allgemeinen zwischen 0,1 und 95 Gew.% Wirkstoff, vorzugsweise zwischen 0,5 und 90 Gew.%.

Die Wirkstoffe können als solche in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001 Gew.%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,02 Gew.%, am Wirkungsort erforderlich.

Beispiel 1:

In einem Reaktionskolben wurden 12,0 g (0,06 Mol) 2-Brom-2-nitro-propandiol-1,3, 3,71 g (0,06 Mol) Borsäure und 4,69 g (0,06 Mol) 2-Mercaptoäthanol in 150 ml Benzol vorgelegt, das Reaktionsgemisch zum Sieden erhitzt und das entstandene Reaktionswasser (3,15 ml) mit einem Wasserabscheider entfernt. Anschließend wurden zur etwas unter den Siedepunkt abgekühlten Lösung 22,93 g (0,06 Mol) Tricyclohexylzinnhydroxid zugegeben, die Reaktionsmischung weiter erwärmt, wobei weitere 1,1 ml Wasser abgetrennt wurden. Nachdem das Reaktionswasser vollständig entfernt worden war, wurde das Lösungsmittel im Vakuum abdestilliert.

Man erhielt 38,0 g 2-[2-Tricyclohexylzinnthia]oxyäthyl-5-brom-5-nitro-1,3,2-dioxaborinan, viskoses Öl.

Beispiel 2:

In einem Reaktionskolben wurden 18,0 g (0,09 Mol) 2-Brom-2-nitro-propandiol-1,3, 5,56 g (0,09 Mol) Borsäure und 33,0 g (0,09 Mol) Triphenylzinnhydroxid in 350 ml Chloroform vorgelegt, das Reaktionsgemisch zum Sieden erhitzt und das entstandene Reaktionswasser mit einem Wasserabscheider entfernt. Nach vollständiger Entfernung des Reaktionswassers (4,9 ml) wurde das Lösungsmittel im Vakuum abdestilliert.

Man erhielt 50,1 g 2-Triphenylzinnoxy-5-brom-5-nitro-1,3,2-dioxaborinan, viskoses Öl.

Beispiel 3:

In einem Reaktionskolben wurden 4,0 g (0,02 Mol) 2-Brom-2-nitro-propandiol-1,3, 1,23 g (0,02 Mol) Borsäure und 11,35 g (0,01 Mol) Bis-(Trineophylzinn)-oxid in 120 ml Toluol vorgelegt, das Reaktionsgemisch zum Sieden erhitzt und das Reaktionswasser mit einem Wasserabscheider entfernt.

Nach Abziehen des Lösungsmittels im Vakuum erhielt man 14 g 2-Trineophylzinnoxy-5-brom-5-nitro-1,3,2-dioxaborinan, viskoses Öl.

$^{1}H$-NMR($CDCl_3$): z = 4,59 ppm, $f_A - f_B$ = 52,4 Hz.

Beispiel 4:

In einem Reaktionskolben wurden 1,84 g (0,0136 Mol) 2-Methyl-2-nitro-propandiol-1,3, 0,84 g (0,0136 Mol) Borsäure und 5,7 g (0,0068 Mol) Bis-(tricyclohexylmethylzinn)oxid in 100 ml Xylol vorgelegt, das Reaktionsgemisch zum Sieden erhitzt und das Reaktionswasser (0,6 ml) mit einem Wasserabscheider entfernt.
Nach Abziehen des Lösungsmittels im Vakuum erhielt man 7,6 g 2-Tricyclohexylmethylzinnoxy-5-methyl-5-nitro-1,3,2-dioxaborinan, viskoses Öl.
$^1H$-NMR($CDCl_3$):z = 4,32 ppm, $f_A - f_B$ = 62,2 Hz.

Beispiel 5:

In einem Reaktionskolben wurden 4,47 g (0,03 Mol) 2-Äthyl-2-nitro-propandiol-1,3, 1,85 g (0,03 Mol) Borsäure und 2,34 g (0,03 Mol) 2-Mercaptoäthanol in 80 ml Benzol vorgelegt, das Reaktionsgemisch zum Sieden erhitzt und das Reaktionswasser (1,9 ml) mit einem Wasserabscheider entfernt. Anschließend wurden zur etwas unter den Siedepunkt abgekühlten Lösung 3,71 g (0,015 Mol) Dibutylzinnoxid zugegeben, die Reaktionsmischung weiter erhitzt, wobei weitere 1,1 ml Wasser entstanden und abgetrennt wurden.
Nach Entfernen des Lösungsmittels erhielt man 10,0 g Bis-/(ß-(5-äthyl-5-nitro-1,3,2-dioxaborinan)-2-yl)-oxy7-äthylthio7-dibutylzinn, viskoses Öl.
$^1H$-NMR($CDCl_3$):z = 4,45 ppm, $f_A$-$f_B$ = 60,4 Hz.

Beispiel 6:

In einem Reaktionskolben wurden 4,0 g (0,02 Mol) 2-Brom-2-nitro-propandiol-1,3, 1,23 g (0,02 Mol) Borsäure und 3,6 g (0,01 Mol) Dioctylzinnoxid in 100 ml Petroläther vorgelegt, das Reaktionsgemisch zum Sieden erhitzt und das Reaktionswasser (0,9 ml) mit einem Wasserabscheider entfernt.
Nach Verdampfen des Lösungsmittels im Vakuum erhielt man 7,8 g Bis-/((5-brom-5-nitro-1,3,2-dioxaborinan)-2-yl)-oxy7-dioctylzinn, viskoses Öl.

0153582

Beispiel 7:

In einem Reaktionskolben wurden 9,71 g (0,015 Mol) Bis-(Dibutylcyclohexylzinn)-oxid, 1,85 g (0,03 Mol) Borsäure und 6,0 g (0,03 Mol) 2-Brom-2-nitropropandiol-1,3 in 100 ml Benzol vorgelegt, das Reaktionsgemisch zum Sieden erhitzt und das Reaktionswasser (1,35 ml) mit einem Wasserabscheider entfernt.
Nach Entfernen des Lösungsmittels im Vakuum erhielt man 16,1 g 2-Cyclohexyldibutylzinnoxy-5-brom-5-nitro-1,3,2-dioxaborinan, viskoses Öl.
$^1H$-NMR($CDCl_3$):z = 4,61 ppm, $f_A$-$f_B$ = 46,8 Hz.

Nach einem der angegebenen Verfahren wurden folgende Verbindungen erhalten:

| Nr. | n | $R_1$ | Y(Z)$_{4-n}$ | | Fp. | $z_{f_A - f_B}$ (ppm) | (Hz) | LM |
|---|---|---|---|---|---|---|---|---|
| 8 | 1 | Br | O | (n-Butyl)$_3$ | Öl | 4,63 | 47,9 | $CDCl_3$ |
| 9 | 1 | Br | O | (Cyclohexyl)$_3$ | Öl | 4,64 | 48,8 | $CDCl_3$ |
| 10 | 1 | $CH_3$ | O | (Cyclohexyl)$_3$ | 65-67°C | 4,34 | 63,5 | $CDCl_3$ |
| 11 | 1 | $CH_3$ | O | (Neophyl)$_3$ | Öl | 4,32 | 66,9 | $CDCl_3$ |
| 12 | 1 | $CH_3$ | $OC_2H_4S$ | (Cyclohexyl)$_3$ | 122-125°C | 4,39 | 63,5 | $CDCl_3$ |
| 13 | 1 | $CH_3$ | $OC_2H_4S$ | (Neophyl)$_3$ | Öl | 4,35 | 65,7 | $CDCl_3$ |
| 14 | 1 | $C_2H_5$ | O | (n-Butyl)$_3$ | Öl | 4,36 | 61,4 | $CDCl_3$ |
| 15 | 1 | $C_2H_5$ | O | (Phenyl)$_3$ | Öl | | | |
| 16 | 1 | $C_2H_5$ | O | (Cyclohexyl)$_3$ | Öl | 4,36 | 63,5 | $CDCl_3$ |
| 17 | 1 | $C_2H_5$ | $OC_2H_4S$ | (n-Butyl)$_3$ | Öl | 4,45 | 64,3 | $CDCl_3$ |
| 18 | 1 | $C_2H_5$ | $OC_2H_4S$ | (Phenyl)$_3$ | Öl | 4,16 | 72,6 | $CDCl_3$ |
| 19 | 1 | $C_2H_5$ | $OC_2H_4S$ | (Cyclohexyl)$_3$ | Öl | 4,42 | 62,9 | $CDCl_3$ |
| 20 | 1 | $C_2H_5$ | $OC_2H_4S$ | (Neophyl)$_3$ | Öl | 4,36 | 66,5 | $CDCl_3$ |
| 21 | 1 | $C_3H_7$ | O | (Cyclohexyl)$_3$ | 72-74°C | 4,37 | 61,9 | $CDCl_3$ |
| 22 | 1 | $C_3H_7$ | $OC_2H_4S$ | (Cyclohexyl)$_3$ | Öl | 4,41 | 61,4 | $CDCl_3$ |
| 23 | 1 | $C_4H_9$ | $OC_2H_4S$ | (Cyclohexyl)$_3$ | Öl | 4,41 | 62,4 | $CDCl_3$ |
| 24 | 1 | $C_5H_{11}$ | $OC_2H_4S$ | (Cyclohexyl)$_3$ | Öl | 4,41 | 61,4 | $CDCl_3$ |
| 25 | 1 | $C_7H_{15}$ | $OC_2H_4S$ | (n-Butyl)$_3$ | Öl | 4,44 | 64,5 | $CDCl_3$ |
| 26 | 1 | $C_7H_{15}$ | $OC_2H_4S$ | (Phenyl)$_3$ | Öl | 4,22 | 71,6 | $CDCl_3$ |

– 12 –

| | | | | | | | | |
|---|---|---|---|---|---|---|---|---|
| 27 | 1 | Phenyl | O | $(Cyclohexyl)_3$ | 55-59°C | 4,81 | 65,5 | $CDCl_3$ |
| 28 | 1 | Br | $OC_2H_4S$ | $(n\text{-}Butyl)_3$ | Öl | | | |
| 29 | 1 | Br | $OC_2H_4S$ | $(Phenyl)_3$ | Öl | 4,44 | 59,2 | $CDCl_3$ |
| 30 | 1 | $C_2H_5$ | O | $(Norbornyl)_3$ | 92-99°C | 4,35 | 62,4 | $CDCl_3$ |
| 31 | 1 | Br | O | $(Norbornyl)_3$ | 139-142°C | 4,62 | 48,5 | $CDCl_3$ |
| 32 | 1 | $C_2H_5$ | O | $(Neophyl)_3$ | Öl | 4,34 | 66,5 | $CDCl_3$ |
| 33 | 1 | $C_3H_7$ | O | $(Neophyl)_3$ | Öl | 4,32 | 66,5 | $CDCl_3$ |
| 34 | 1 | Br | $OC_2H_4S$ | $(Neophyl)_3$ | Öl | 4,60 | 50,0 | $CDCl_3$ |
| 35 | 1 | Br | O | $(Cyclohexylmethyl)_3$ | Öl | 4,60 | 47,5 | $CDCl_3$ |
| 36 | 1 | $CH_3$ | O | $(Butyl)_2Cyclohexyl$ | Öl | 4,33 | 60,4 | $CDCl_3$ |
| 37 | 1 | Br | $OC_6H_4S$ | $(Cyclohexyl)_3$ | Öl | 4,38 | 41,0 | $CDCl_3$ |
| 38 | 1 | $CH_3$ | $OC_6H_4S$ | $(Cyclohexyl)_3$ | Öl | 4,36 | 60,2 | $CDCl_3$ |
| 39 | 1 | Br | $OC_6H_4S$ | $(Phenyl)_3$ | Öl | 4,46 | 51,0 | $CDCl_3$ |
| 40 | 2 | Br | O | $(Phenyl)_2$ | Öl | | | |
| 41 | 2 | $C_2H_5$ | O | $(Phenyl)_2$ | Öl | | | |
| 42 | 2 | $C_2H_5$ | O | $(n\text{-}Octyl)_2$ | Öl | | | |
| 43 | 2 | Br | $OC_2H_4S$ | $(n\text{-}Butyl)_2$ | Öl | | | |
| 44 | 2 | Br | $OC_2H_4S$ | $(n\text{-}Octyl)_2$ | Öl | | | |
| 45 | 2 | $C_2H_5$ | $OC_2H_4S$ | $(n\text{-}Octyl)_2$ | Öl | 4,43 | 59,4 | $CDCl_3$ |
| 46 | 2 | Br | $OC_2H_4S$ | $(Phenyl)_2$ | Öl | 4,32 | 16,2 | $CDCl_3/CD_3OD$ |
| 47 | 2 | $C_2H_5$ | $OC_2H_4S$ | $(Phenyl)_2$ | Öl | | | |
| 48 | 1 | $CH_3$ | O | $(Neopentyl)_3$ | 64-68°C | | | |
| 49 | 1 | iso-Butyl | O | $(Phenyl)_3$ | 125-132°C | 4,33 | 63,1 | $CDCl_3$ |
| 50 | 1 | $CH_3$ | O | $(Norbornyl)_3$ | 147-152°C | | | |

Beispiel 51:

20 Gew.Teile der Verbindung nach Beispiel 2 wurden mit 70 Gew.Teilen Attapulgit und 10 Gew.Teilen Na-Oleylmethyltaurid (ca. 33%ig) vermengt, in einer Stiftmühle feinst vermahlen, wodurch ein Spritzpulver erhalten wurde. Durch Einrühren in Wasser wurde eine für die Applikation geeignete Suspension gebildet.

Beispiel 52:

50 Gew.Teile der Verbindung 15 wurden mit 41 Gew.Teilen einer hochdispersen Kieselsäure, 6 Gew.Teilen Na-Oleylmethyltaurid (ca. 33%ig) und 3 Gew.Teilen Na-Diisobutylnaphthalinsulfonat vermischt und anschließend in einer Kugelmühle 4 Stunden vermahlen, wodurch ein Spritzpulver entstand. Durch Einrühren in Wasser wurde eine für die Applikation geeignete Suspension gebildet.

Beispiel 53:

20 Gew.Teile der Verbindung nach Beispiel 1 wurden in Methylenchlorid gelöst, mit 70 Gew.Teilen Attapulgit versetzt und das Lösungsmittel verdampft. Der Rückstand wurde mit 10 Gew.Teilen Na-Oleylmethyltaurid (ca. 33%ig) versetzt und in einer Kugelmühle 4 Stunden vermahlen, wodurch ein Spritzpulver entstand. Durch Einrühren in Wasser wurde eine für die Applikation geeignete Suspension gebildet.

Beispiel 54:

20 Gew.Teile der Verbindung 17 wurden in 35 Gew.Teilen Dimethylformamid, 35 Gew.Teilen Xylol und 10 Gew.Teilen Alkylarylsulfonat in Mischung mit Polyoxyäthylen-Sorbitan-Tallölester versetzt, worauf ein Emulsionskonzentrat entstand. Durch Einrühren in Wasser wurde eine für die Applikation geeignete Emulsion gebildet.

Beispiel A:

In vitro-Prüfungen

Bei in vitro-Prüfungen wurden Agar-Nährböden folgender Zusammensetzungen verwendet:

Nährboden I: Bestehend aus Agar und Hafermehl

Nährboden II: Bestehend aus Agar, Biomalz, Holzmehl und Peptone

Nährboden III: Bestehend aus Agar, Biomalz und Peptone
= 5%iger Malzextraktagar

Nährboden IV: Bestehend aus Agar, Glucose, Haferextrakt und Peptone.

Von den Wirkstoffformulierungen wurden wäßrige Dispersionen von 0,5 %, 0,2 %, 0,04 %, 0,008 % und 0,0016 % hergestellt. Filterpapierplättchen von 5 mm Durchmesser wurden darin getränkt und anschließend auf den beimpften Nährboden gelegt. Derart wurden Botrytis cinerea, Aspergillus niger, Alternaria tenuis, Penicillium glaucum, Trichoderma viridae und Xanthomonas ssp. geprüft. Bei der Prüfung von Merulius lacrimans, Polystictus cinnabarinus, Polyporus vaporarius, Coniophora puteana, Poria placenta und Rhizoctonia solani wurden die wäßrigen Wirkstoffdispersionen direkt dem auf 40°C abgekühlten Nährboden zugegeben und auf Konzentrationen von 0,5 %, 0,2 %, 0,04 %, 0,008 % und 0,0016 % eingestellt.

Im Nährboden I wurden Alternaria tenuis und Trichoderma viridae,

im Nährboden II wurden Merulius lacrimans, Polystictus cinnabarinus und Polyporus vaporarius,

im Nährboden III wurden Botrytis cinerea, Aspergillus niger, Coniophora puteana, Penicillium glaucum, Poria placenta und Rhizoctonia solani und

im Nährboden IV wurde Xanthomonas ssp.

gezogen.

In einer Konzentration von 0,0016 % wurde z.B. von folgenden Verbindungen das Wachstum folgender Kulturen verhindert:

Alternaria tenuis von Verbindung 14
Aspergillus niger von den Verbindungen 10, 16, 17 und 19
Botrytis cinerea von Verbindung 14
Penicillium glaucum von den Verbindungen 14 und 15
Polyporus vaporarius von Verbindung 14
Merulius lacrimans von Verbindung 14
Trichoderma viridae von den Verbindungen 8, 14, 24 und 25 und
Xanthomonas ssp. von Verbindung 14.
In einer Konzentration von 0,008 % verhinderte beispielsweise die Verbindung 14 das Wachstum von Rhizoctonia solani.

Beispiel B:

Wirkung der Substanzen gegen
Pseudocercosporella herpotrichoides im Plattentest

Filterpapierscheiben wurden in wäßrigen Dispersionen der formulierten Wirkstoffe getränkt, die 0,2 % Wirkstoff enthielten. Diese Scheiben wurden dann auf Hafermehl-Agar-Nährböden gelegt, die vorher mit Myzel von Pseudocercosporella herpotrichoides beimpft worden waren. Anschließend wurden die Schalen bei 5°C drei Tage lang bebrütet und dann bei künstlicher Beleuchtung bei 22°C gehalten. Nach 12 Tagen war die Kontrolle gänzlich vom Pilzmyzel überwachsen. Zu diesem Zeitpunkt wurde augewertet, indem der Durchmesser des Hemmhofes bestimmt wurde. Ein Hemmhofdurchmesser von größer als 1 cm wurde beispielsweise bei den Verbindungen 14, 28 und 25 gemessen.

Beispiel C:

Einfluß der Wirkstoffe auf das Wachstum
der Alge Chlorella fusca

Zu 70 ml Algensuspension entsprechend 0,1 mg Algen/1 ml Nährlösung wurde die zu prüfende Substanz, gelöst in Aceton, zugegeben. Die Algensuspensionen wurden 48 Stunden lang bei künstlicher Beleuchtung und Belüftung gehalten. Ausgewertet wurde der Prozentwert (P) = Algenkonzentration (in mg) in der Testlösung im Verhältnis zur Kontrolle (= 100%). Bei einer Wirkstoffkonzentration von 5 pmm ergaben sich beispielsweise bei der Verbindung nach Beispiel 2 und den Verbindungen 15 und 26 Prozentwerte von kleiner als 1.

Beispiel D:

Wirkung der Substanzen gegen Plasmopora viticola
an Weinstecklingen im Gewächshaus

Junge Topfreben der Sorte "Grüner Veltliner" mit 3 bis 6 Laubblättern wurden bis zur Tropfnässe mit einer 0,01%igen wäßrigen Dispersion des formulierten Wirkstoffes besprüht. Bis zum Trocknen des Spritzbelages verblieben die Pflanzen im Gewächshaus bei 20$^{0}$C und rund 70 % Luftfeuchtigkeit. Anschließend wurden die Rebpflanzen mit einer wäßrigen Sporensuspension des Pilzes Plasmopora viticola inokuliert. Die derart infizierten Pflanzen wurden anschließend für 48 Stunden in einer Feuchtkammer bei 18$^{0}$C und 95 % Luftfeuchtigkeit im Dunkeln gehalten und kamen dann in Klimakammern mit hoher Luftfeuchtigkeit. Nach Ausbruch der Krankheit an den Blättern der Kontrollpflanzen wurde der Befall mit Plasmopora viticola festgestellt. Es konnte beispielsweise die Verbindungen 26 und 49 den Ausbruch der Krankheit zu 100 % verhindern.

Beispiel E:

Wirksamkeit gegen Helminthosporium gramineum
bei Anwendung als Beizmittel (Freilandversuch)

Mit Streifenkrankheit natürlich infiziertes Gersten-Saatgut (Wintergerste) wurde mit 30 g Wirkstoff/100 kg Saatgut sorgfältig gebeizt und auf 2 m$^2$ großen Parzellen in 2 Wiederholungen angebaut. Die Bonitierung erfolgte durch Zählen der kranken Pflanzen zum Zeitpunkt des Schossens.

$$\text{Wirkungsgrad in \%} = \frac{\text{krank in Unbehandelt - krank in behandelt}}{\text{krank in Unbehandelt}} \times 100$$

Dabei erreichten beispielsweise die Verbindungen 8, 14 und 28 einen Wirkungsgrad von 100 %.

Beispiel F:

Wirkung gegen Septoria apii an Selleriepflanzen

Sellerietopfpflanzen im 5- bis 6Blattstadium wurden mit den wäßrigen Dispersionen der formulierten Prüfsubstanzen allseits benetzt. Nach dem Trocknen des Spritzbelages wurden die Pflanzen mit von infizierten Pflanzen erhaltenen Sporen inokuliert, indem diese als Suspension versprüht wurden. Anschließend wurden die Pflanzen in einem Klimaraum bei ca. 18°C und möglichst hoher relativer Luftfeuchtigkeit inkubiert und nach 14 Tagen der Pilzbefall festgestellt. In einer Konzentration von 0,005 % konnten dabei die Verbindung nach Beispiel 2 und die Verbindungen 8, 14, 17 und 28 das Ausbrechen der Krankheit zu 100 % verhindern.

Beispiel G:

Kontakt-Wirkung gegen Tetranychus urticae (adult)

Die Primärblätter der Buschbohne (Phaseolus vulgaris) wurden 48 Stunden vor Versuchsbeginn mit einem infestierten Blatt aus der Massenzucht von Tetranychus urticae belegt. Diese Pflanzen wurden dann mit wäßrigen Dispersionen der formulierten Wirkstoffe bis zur Tropfnässe besprüht und anschließend bei rund 23°C im Gewächshaus stehen gelassen. 24 Stunden nach der Behandlung wurden alle beweglichen Stadien unter dem Binokular auf lebende und tote Individuen ausgewertet. Bei einer Konzentration von 0,001 % zeigten beispielsweise die Verbindung nach Beispiel 4 und die Verbindungen 10, 16, 19, 23, 24, 30, 37 und 48 eine 100%ige Wirkung.

Beispiel H:

Kontakt-Wirkung gegen Aphis fabae auf Sellerie

Mit Aphis fabae gut besiedelte Sellerietopfpflanzen (Apium graveolens) wurden mit einer wäßrigen Dispersion des formulierten Wirkstoffes bis zur Tropfnässe besprüht und anschließend bei rund 23°C im Gewächshaus aufgestellt. 24 Stunden nach der Behandlung wurde unter dem Binokular auf tote und lebende Individuen ausgewertet. Bei einer Wirkstoffkonzentration von 0,05 % erreichten beispielsweise die Verbindungen 8,14,17,28 und 48 einen 100%igen Bekämpfungserfolg.

Beispiel I:

Insektizide Kontaktwirkung
Bestimmung der LD50-Werte an
Drosophila melanogaster, Musca domestica, Blattella germanica und Calandra granaria

Die Wirkstoffe wurden in den angegebenen Konzentrationen in Aceton gelöst und in Petrischalen von 10 cm Durchmesser gesprüht. Nach dem Antrocknen des Spritzbelages wurden die Individuen zugegeben und die Petrischalen umgestürzt auf Filterpapier gelegt. Nach 24 Stunden bei Drosophila melanogaster und Musca domestica und nach 48 Stunden bei Blattella germanica und Calandra granaria wurden die toten und lebenden Individuen ausgezählt.

In der Tabelle sind die Wirkstoffe und jene Konzentrationen angegeben, bei der der LD50-Wert erreicht wurde.

| Nr. | Blattella germanica | Calandra granaria | Drosophila melanogaster | Musca domestica |
|---|---|---|---|---|
| 17 | 0,05 % | 0,075 % | 0,05 % | - |
| 14 | 0,25 % | - | 0,025 % | - |
| 28 | 0,25 % | 0,1 % | 0,05 % | - |
| 8 | 0,5 % | 0,25 % | 0,05 % | - |
| 10 | - | - | 0,1 % | - |
| 9 | - | - | 0,25 % | - |
| 21 | - | - | 0,25 % | 0,1 % |
| 16 | - | - | - | 0,1 % |

Beispiel J:

Wirkung gegen Larven von Plutella xylostella

Kohlblätter wurden für 10 Sekunden in die wäßrige Lösung des formulierten Wirkstoffes getaucht und nach dem Antrocknen des Belages zusammen mit den Larven von Plutella xylostella in Petrischalen gegeben. Nach 7 Tagen wurden die toten und lebenden Individuen ausgezählt. Dabei zeigte beispielsweise die Verbindung Nr.9 folgende Werte:

| Konzentration (ppm) | % Mortalität nach 7 Tagen |
|---|---|
| 250 | 100 |
| 125 | 95 |
| 62,5 | 95 |
| 31,3 | 55 |

P a t e n t a n s p r ü c h e :

1. 1,3,2-Dioxaborinane der allgemeinen Formel

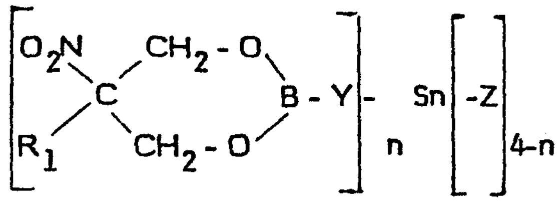

(I) ,

in welcher $R_1$ einen geradkettigen oder verzweigten Alkylrest mit 1 bis 20 Kohlenstoffatomen, einen Phenylrest oder Brom, Y einen 1-Oxy-2-thioäthylrest, einen 1-Oxy-4-thiophenylrest oder Sauerstoff, jeder Rest Z unabhängig von den anderen einen geradkettigen oder verzweigten Alkylrest mit 1 bis 20 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet.

2. 1,3,2-Dioxaborinane der allgemeinen Formel I gemäß Anspruch 1, dadurch gekennzeichnet, daß $R_1$ einen geradkettigen oder verzweigten Alkylrest mit 1 bis 10 Kohlenstoffatomen, einen Phenylrest oder Brom, Y einen 1-Oxy-2-thioäthylrest, einen 1-Oxy-4-thiophenylrest oder Sauerstoff, jeder Rest Z unabhängig von den anderen einen geradkettigen oder verzweigten Alkylrest mit 1 bis 10 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet.

3. Verfahren zur Herstellung von 1,3,2-Dioxaborinanen der allgemeinen Formel

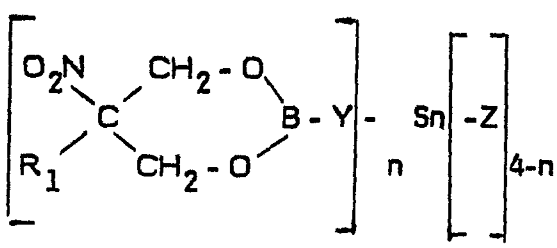

(I) ,

in welcher $R_1$ einen geradkettigen oder verzweigten Alkylrest mit 1 bis 20 Kohlenstoffatomen, einen Phenylrest oder Brom, Y einen 1-Oxy-2-thio-

äthylrest, einen 1-Oxy-4-thiophenylrest oder Sauerstoff, jeder Rest Z unabhängig von den anderen einen geradkettigen oder verzweigten Alkylrest mit 1 bis 20 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet, dadurch gekennzeichnet, daß man ein Diol der allgemeinen Formel

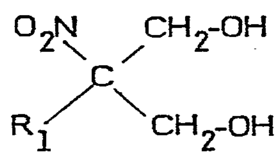

(II) ,

in welcher $R_1$ die oben angegebene Bedeutung hat, mit der äquivalenten Menge von Borsäure oder deren Anhydriden und gegebenenfalls mit der äquivalenten Menge von 2-Mercaptoäthanol oder von 4-Mercaptophenol bei einer Temperatur von 30 bis 180°C gegebenenfalls in einem unter Reaktionsbedingungen inerten Verdünnungsmittel unter Abspaltung von Wasser umsetzt und das Reaktionsprodukt, falls n = 1 ist, mit der äquivalenten Menge einer Verbindung der allgemeinen Formel

$$HO - Sn(-Z)_3 \qquad (III),$$

in der Z die oben angegebene Bedeutung hat oder deren Anhydrid, und falls n = 2 ist, mit der äquivalenten Menge einer Verbindung der allgemeinen Formel

$$O = Sn(-Z)_2 \qquad (IV),$$

in der Z die oben angegebene Bedeutung hat, unter weiterer Wasserabspaltung bei einer Temperatur von 30 bis 180°C umsetzt und anschließend gegebenenfalls das inerte Verdünnungsmittel entfernt.

4. Verfahren nach Anspruch 2 zur Herstellung von 1,3,2-Dioxaborinanen der allgemeinen Formel I, in der $R_1$, Z und n die in Anspruch 2 angegebene Bedeutung haben und Y Sauerstoff bedeutet, dadurch gekennzeichnet, daß alle Reaktionspartner in dem inerten Verdünnungsmittel vorgelegt werden,

und das entstehende Reaktionswasser durch Azeotropdestillation entfernt wird.

5. Biozide Mittel, gekennzeichnet durch einen Gehalt an mindestens einem 1,3,2-Dioxaborinan der allgemeinen Formel

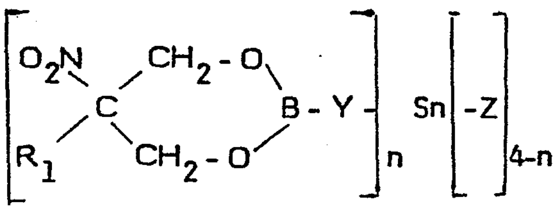

(I) ,

in welcher $R_1$ einen geradkettigen oder verzweigten Alkylrest mit 1 bis 20 Kohlenstoffatomen, einen Phenylrest oder Brom, Y einen 1-Oxy-2-thioäthylrest, einen 1-Oxy-4-thiophenylrest oder Sauerstoff, jeder Rest Z unabhängig von den anderen einen geradkettigen oder verzweigten Alkylrest mit 1 bis 20 Kohlenstoffatomen, einen Cycloalkyl-, Bicycloalkyl- oder Cycloalkylalkylrest, einen Phenylrest oder einen Phenylalkylrest und n die Zahl 1 oder 2 bedeutet.

6. Fungizide und algizide Mittel der allgemeinen Formel I nach Anspruch 5, dadurch gekennzeichnet, daß die Reste $R_1$, Y, Z und die Zahl n die in Anspruch 5 angegebene Bedeutung haben.

7. Insektizide und akarizide Mittel der allgemeinen Formel I nach Anspruch 5, dadurch gekennzeichnet, daß die Reste $R_1$, Y und die Zahl n die in Anspruch 5 angegebene Bedeutung haben und daß jeder Rest Z unabhängig von den anderen einen geradkettigen oder verzweigten Alkylrest mit 1 bis 20 Kohlenstoffatomen, einen Cycloalkyl-, Bicycloalkyl-, Cycloalkylalkyl- oder einen Phenylalkylrest bedeutet.

8. Verfahren zur Bekämpfung von Schadorganismen, dadurch gekennzeichnet, daß man 1,3,2-Dioxaborinane der allgemeinen Formel I, in welcher die Reste $R_1$, Y, Z und die Zahl n die in Anspruch 5 angegebene Bedeutung haben, auf Schadorganismen oder deren Lebensraum einwirken läßt.

9. Verwendung von 1,3,2-Dioxaborinanen der allgemeinen Formel I, in welcher die Reste $R_1$, Y, Z und die Zahl n die in Anspruch 5 angegebene Bedeutung haben, zur Bekämpfung von Schadorganismen.

10. Verfahren zur Herstellung von bioziden Mitteln, dadurch gekennzeichnet, daß man 1,3,2-Dioxaborinane der allgemeinen Formel I, in welcher die Reste $R_1$, Y, Z und die Zahl n die in Anspruch 5 angegebene Bedeutung haben, mit Streckmitteln und/oder oberflächenaktiven Mitteln vermischt.

O.Z.750
19.12.1984